(19) Europäisches Patentamt / European Patent Office / Office européen des brevets

(11) EP 4 663 210 A1

(12) **EUROPEAN PATENT APPLICATION**

(43) Date of publication:
**17.12.2025 Bulletin 2025/51**

(21) Application number: **24382629.4**

(22) Date of filing: **11.06.2024**

(51) International Patent Classification (IPC):
***A61L 27/14*** *(2006.01)* ***C12N 5/00*** *(2006.01)*
***C12N 5/0783*** *(2010.01)*

(52) Cooperative Patent Classification (CPC):
**C12N 5/0062; C12N 5/0068; C12N 5/0636**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Consejo Superior de Investigaciones Científicas (CSIC)**
  **28006 Madrid (ES)**
- **Fundació de Recerca Clínic Barcelona-Institut d'Investigacions Biomèdiques August Pi I Sunyer**
  **08036 Barcelona (ES)**

(72) Inventors:
- **Guasch Camell, Judit**
  **08193 Bellaterra (Barcelona) (ES)**
- **Castellote Borrell, Miquel**
  **08193 Bellaterra (Barcelona) (ES)**
- **Merlina, Francesca**
  **08193 Bellaterra (Barcelona) (ES)**
- **Faraudo Gener, Jordi**
  **08193 Bellaterra (Barcelona) (ES)**
- **Domingo Cabases, Marc**
  **08193 Bellaterra (Barcelona) (ES)**
- **Guedan, Sonia**
  **08036 Barcelona (ES)**

(74) Representative: **Pons IP**
**Glorieta Rubén Darío 4**
**28010 Madrid (ES)**

(54) **INVERSE OPAL HYDROGEL AND ITS USE IN IMMUNOTHERAPY**

(57) The present invention relates to an inverse opal (IOPAL) hydrogel formed with poly(ethylene)glycol (PEG) covalently bonded to heparin (Hep) and having a storage shear modulus (G') between 2.5 and 4 kPa. Said hydrogel with improved mechanical and structural properties has demonstrated to be highly effective in CAR T cell expression and, therefore, in CAR T adoptive cell therapy.

EP 4 663 210 A1

## Description

The invention relates to an inverse opal (IOPAL) hydrogel formed with poly(ethylene)glycol (PEG) covalently bonded to heparin (Hep) with improved mechanical and structural properties to promote CAR T cell expression. The invention also relates to the use of said hydrogel in immunotherapy as well as to the procedure, based in inverted colloidal crystal technique, for preparing the same.

## BACKGROUND ART

Adoptive cell therapy (ACT) is a novel immunotherapy that is achieving unprecedented results in oncology, including long-term remissions of relapsed and refractory cancers. It consists of harnessing the power of the immune system by using immune cells, usually autologous T cells, as "living" drugs after ex vivo conditioning. In particular, T cells are harvested from the patients, genetically modified or selected, and then expanded in vitro. Once an adequate number of therapeutic cells is reached, they are re-infused into the patients to mediate cancer cell destruction. A major challenge of ACT is to manufacture the large amounts of therapeutic T cells required to obtain long-lasting responses.

Chimeric antigen receptor (CAR)-T therapy is an ACT approach that consists of genetically modifying T cells to express an artificial receptor, the CAR, that selectively targets cancer cells. Specifically, a viral vector is used for the transduction of T cells with the CAR of interest during their expansion in vitro. Anti-CD19 CAR T therapies against cancerous B cells occurring in leukemia and lymphoma have been approved by the FDA and EMA and are currently used in clinical practice with good results. Despite such success, some challenges lay ahead, such as the optimization of the costs related to these therapies. Currently, viral transduction and cell expansion steps are two of the highest costs involved in CAR-T therapy. Therefore, their optimization is key for CAR-T therapy to become a broadly used therapy (Guedan, S. et al.Annu. Rev. Immunol. 37, 145-171 (2018)).

*In vivo*, resting T cells are activated in the lymph nodes upon their interaction with antigen presenting cells (APCs) through the immunological synapse. Once activated, T cells start to proliferate, aided by the architectural and biological cues that the lymph nodes offer. However, *ex vivo* T cell expansion methods have so far only focused on mimicking the activation provided by APCs (Wong, W. K. et al. Eng. Regener. 2, 70-81 (2021); Isser, A. et al. Biomaterials 268, 120584 (2021)). Even nanoscale control of the spatial distribution of such signals has been explored to efficiently mimic the IS. Nevertheless, the most common approach and current gold standard for ex *vivo* polyclonal activation of T cells is the use of polymeric magnetic beads coated with anti-CD3 and anti-CD28 antibodies (e.g. Dynabeads, ThermoFisher Scientific) (Trickett, A. & Kwan, Y. L. T J. Immunol. Methods 275, 251-255 (2003)). Dynabeads offer a potent T cell activation, but they alone fail to reproduce the complexity of the *in vivo* environment in which T cells activate and proliferate.

To tackle this deficiency, some efforts have been made to engineer materials that can mimic the properties of lymph nodes. Among them, we showed how hydrogels made of poly(ethylene glycol) crosslinked with heparin (PEG-Hep) could improve the expansion of primary human CD4+ T cells by providing adequate mechanical and biochemical stimuli to the cells (Pérez del Rio, E. et al. Biomaterials 259, 120313 (2020); EP3913048A1). In a more advanced study, a second generation of PEG-Hep hydrogels were engineered with precise pore size and enlarged interconnectivity (Santos, F. et al. Biomater. Sci. 10, 3730-3738 (2022); EP4252789A1) through the inverse OPAL (IOPAL) technique. These IOPAL hydrogels showed improved viability and proliferation of primary human CD4+ T cells compared to bulk hydrogels and suspension systems.

CAR-T manufacturing involves a genetic modification of T cells, which is commonly performed with lentiviral vectors, *i.e.* lentiviruses (LV) used to genetically modify cells. LV have the ability to infect and express genes in both dividing and nondividing cells, giving them the possibility to become endogenous (hereditary). For gene delivery purposes, different generations of LV have been designed to improve their safety and transduction efficiency (Dull, T. et al. J. Virol. 72, 8463-8471 (1998)). An important modification of lentiviral vectors is the change of the envelope protein from HIV-1 gp120, which is specific for the CD4 receptor, for VSV-G, a glycoprotein that recognizes a much more universal receptor across cell types called LDL-R. However, lentiviral vectors featuring VSV-G are unable to infect unstimulated T cells, as they lack the LDL-R (Roche, S.Cell. Mol. Life Sci. 65, 1716-1728 (2008)).

The efficiency of transduction of the CAR gene into T cells depends on the multiplicity of infection (MOI), which is the number of transducing lentiviral particles per cell (Finck, A. V., Blanchard, T., Roselle, C. P., Golinelli, G. & June, C. H. Engineered cellular immunotherapies in cancer and beyond. Nat. Med. 28, 678-689 (2022)). The higher the MOI, the more efficient the transduction. However, high MOI can result in tonic signaling, which is an overactivation of T cells produced by CARs interacting with each other. To correctly determine the MOI, the concentration of a batch of lentiviral particles is experimentally determined by tittering the virus following a standard protocol. It has been studied that in a suspension system, the number of viral particles that reach a cell follows a Poisson distribution. The use of a different culture system, such as PEG-heparin hydrogels, might change this distribution by breaking the randomness of the events of viral particles and cells finding each other. In the present invention, novel IOPAL PEG-Hep hydrogels were developed with optimized mechanical and structural properties to enhance not only CAR T cell proliferation, but also and more important, CAR T cell

expression, with is essential to improve CAR T adoptive cell therapy.

## SUMMARY OF THE INVENTION

In the present invention refers to novel IOPAL PEG-Hep hydrogels with optimized mechanical and structural properties to resemble artificial lymph nodes and improve primary human CAR-T cell (chimeric antigen receptor T cell) manufacture. In particular, enhanced (CD19) CAR expression and cell proliferation were obtained with stiff and heparin-containing hydrogels, as experimentally demonstrated and explained through modelling. CD4+ T cells have recently been identified as capable of producing MHC-II-mediated cytotoxicity, and, while not so efficient as CD8+ T cells at, they possess the advantages of combining cytotoxicity with chemokine secretion, circumventing typical MHC-I related tumor resistance, and an improved longer-term effect.

Then, the IOPAL PEG-Hep hydrogels of the present inventions are optimal for use in CAR T adoptive cell therapy. In CAR T adoptive cell therapy, T cells are harvested and purified from the patient's blood, and activated in vitro. For the cells to be able to recognize cancer cells specifically, a lentiviral vector is used to transduce the T cells with the genetic information necessary to express the CAR receptor. This artificially engineered receptor recognizes an epitope present in the surface of a cancer-specific cell type. In our project, we use an anti-CD19 CAR, which is specific for leukemia and lymphoma. In the manufacturing of CAR T cell products, it is necessary to achieve a sufficient expansion of the cell population, but also ensure that as many cells as possible are expressing the CAR receptor, since only those cells are able to recognize and therefore target the cancerous leukemia and lymphoma cells.

In this invention, CAR T cells, which are the clinically relevant cells for CAR adoptive cell therapies have been studies. In this case, proliferation is important, but also key is to achieve a cell population that is effective in targeting the tumor cells, i.e. it is equipped with the CAR receptor. Therefore, the CAR expression of T cells is considered very valuable.

The inventors of the present invention have surprisingly found that IOPAL hydrogel stiffness (defined by the PEG wt% and storage shear modulus(G')), together with the IOPAL architecture, are the keys for the enhancement in CAR expression of T cells, in addition to enhance cell proliferation.

As demonstrated in the examples, the most surprising effect was an enhancement of CAR expression when IOPAL hydrogels have a storage shear modulus (G') between 2.6 and 3.1 kPa and, preferably, 6wt% of functionalized PEG multi-arm star polymer. Within hydrogels of 6wt%, the ones with 120 μm pores were the most suitable ones. Moreover, we saw that the presence of heparin was important (non-heparin hydrogels worked worse) and that a 120 μm pore size was advantageous compared to smaller pore sizes.

Then, a first aspect of the present invention related to a hydrogel, hereinafter the hydrogel of the invention, comprising a functionalized PEG multi-arm star polymer covalently combined with heparin in the form of inverse opal scaffold having a storage shear modulus (G') between 2.5 and 4 kPa, as measured by small-amplitude oscillatory shear (SAOS) rheology.

The equipment used in the present invention for measuring storage shear modulus (G') was a Rheometer HAAKE RheoStress RS600 (Thermo Electron Corporation, USA) with a 10 mm diameter rotor.

As it is known in the state of the art, "opal" refers to the crystalline array of close-packed spheres and "inverse" refers to the fact that this array is the negative space, or the resulting pores. Then, an inverse opal structure, or inverted colloidal crystal, is the negative replica of the opal structure, in which the solid spheres are replaced by air phase forming pores whereas the space between spheres is filled with a new material (the hydrogel material in the present invention). Consequently, an inverse opal structure is a porous structure that possess an ordered array of uniform pores. In this invention, the pores have dimensions on the micrometre scale.

These structures are obtained by the inverse opal technique, also called inverted colloidal crystal technique. As known, this technique is based on the use of a colloidal crystal template to subsequently create homogenous (micro)pores in the material upon the elimination of the template. The technique comprises the next steps:

- adding a solution of spheres into a template,
- evaporating the solvent comprising the spheres,
- adding a solution comprising the hydrogel components into the template,
- dissolving the spheres in a solution.

As used herein, the term "hydrogel" refers to a 3D network of a polymeric material which exhibits the ability to swell in water and to retain a significant portion of water within its structure without dissolving.

In a preferred embodiment, the hydrogel of the present invention has a storage shear modulus (G') between 2.6 and 3.5 kPa, more preferably between 2.6 and 3.1 kPa and even more preferably, 3.1 kPa.

In a preferred embodiment, the hydrogel of the present invention has an average pore diameter between 110 and 150 μm, preferably 120 as measured by environmental scanning electron microscopy (ESEM).

The terms "poly(ethylene glycol)", "polyethyleneglycol" or "PEG" or "polyethylene oxide" or "polyoxyethylene"

are considered equivalents and can be used interchangeably herein.

PEG is a hydrophilic and biocompatible polymer. It offers the possibility to design scaffolds with very well-controlled structural and mechanical properties.

In a preferred embodiment, the molecular weight (MW) of the functionalized PEG multi-arm star polymer is between 5 and 20 kDa, more preferably, between 7 and 15 KDa, even more preferably, 10 kDa.

The term "multi-arm star" used herein refers to star-shaped polymers, which are the simplest class of branched polymers with a general structure consisting of at least three linear chains connected to a central core, wherein said core or the centre of the polymer can be an atom, molecule, or macromolecule and the chains, or "arms", consist of variable-length organic chains. Star-shaped polymers in which the arms are all equivalent in length and structure are considered homogeneous, whereas the ones with variable lengths and structures are considered heterogeneous.

In the present invention, the use of PEG multi-arm star polymer is essential to form the hydrogel, otherwise a hydrogel in the form of inverse opal scaffold cannot be formed adequately to be useful in CAR T cell expression.

Preferably, the PEG multi-arm star polymer is functionalized with reactive end groups including, without limitation, N-hydroxysuccinimide ester, thiol, carboxyl, carbonyl, primary amine, aldehyde, anhydride, epoxide, maleimide, pyridyl disulfide, amines, hydrazides, isocyanate, sulfonyl chloride, fluorobenzene, imidoester, haloacetyl, vinylsulfone, carbodiimide, alkoxyamines, or iodoacetyl.

More preferably, the PEG-multi-arm polymer in the hydrogel of the invention is functionalized with thiol groups forming thiolated PEG or methoxy PEG thiol or methoxypolyethylene glycol thiol or mPEG thiol, wherein these terms are considered equivalents and can be used interchangeably herein (represented as PEG-SH).

In another preferred embodiment, the functionalized PEG multi-arm star polymer is a functionalized PEG four-arm star polymer; and more preferably is a thiol-functionalized PEG four-arm star polymer of formula:

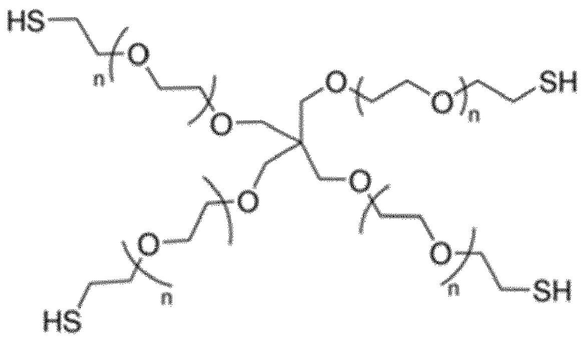

The concentration of the functionalized PEG multi-arm star polymer in the hydrogel of the invention comprising a certain range. In a preferred embodiment of the invention, the concentration of the functionalized PEG multi-arm star polymer in the hydrogel of the invention ranges between 5%wt to 8%wt on the total weight percentage of hydrogel, more preferably, 6% to 8%wt and, even more preferably, 6%wt.

The terms "% by weight" or "% wt" or "%w/w" are considered equivalents and can be used interchangeably herein and refer to the weight percentage.

The concentration of PEG multi-arm star polymer is a relevant parameter to obtain hydrogels with the desired stiffness. A concentration of the functionalized PEG multi-arm star polymer in the hydrogel of the invention between 5%wt to 8%wt on the total weight percentage of hydrogel is adequate.

Further, the hydrogel of the invention comprises heparin. Heparin is a naturally occurring highly sulphated glycosaminoglycan (GAG). Therefore, it has combined advantages such as being a biomolecule present in the human body, presenting a very negative charge that allows for electrostatic interactions with molecules of interest, and having the possibility to functionalize it with functional groups such as maleimide for different applications, like supramolecular chemistry or materials science.

A non-fractionated heparin is preferably used in the present invention. Non-fractionated heparin, also referred as unfractionated heparin or UFH, refers to a type of heparin that has not been treated or subjected to purification to obtain low molecular weight heparin.

The advantages of using non-fractionated heparin over further processed heparins comprise a lower cost and the ease to have a steady supply, both things derived from the simpler production process.

In another more preferred embodiment, the hydrogel of the invention comprises functionalized heparin, more preferably, maleimide-functionalized heparin, hereinafter Hep-Mal.

The use of Hep-Mal allows to form hydrogels through the reaction of maleimide with thiols that can be incorporated to the PEG molecules.

The maleimide-thiol reaction, which is a Michael reaction, offers many advantages versus other types of

reactions used for hydrogel formation, such as the well-known acrylate cross-linking which requires UV light. First, the maleimide-thiol reaction is a "Click Chemistry" strategy, which means that is fast, results in a high yield, and happens in mild and biocompatible conditions. Secondly, it does not require UV light, which is harmful for cell viability as it introduces DNA damage. Finally, maleimide offers a very versatile anchoring point for biological molecules, as it can be crosslinked with available thiol groups through Cys amino acids of proteins.

The functionalized PEG multi-arm star polymer is covalently combined with Hep-Mal forming a PEG-Hep hydrogel. The PEG-Hep hydrogel is formed through a reaction between the maleimide of the functionalized heparin and the reactive end groups of the functionalized PEG-multi-arm, that result in a covalent crosslink and the consequent gelation. The reactive end group of the functionalized PEG multi-arm have been previously described herein and apply equally to this embodiment.

In a preferred embodiment of the invention, the concentration of heparin, or of functionalized heparin in case it is functionalized, in the hydrogel of the invention ranges between 1%wt to 10%wt on the total weight percentage of hydrogel.

In another preferred embodiment of the hydrogel of the invention, the concentration of heparin, or of functionalized heparin in case it is functionalized, is between 2%wt to 6%wt on the total weight percentage of hydrogel, and even more preferably, is 4.5%wt.

In another preferred embodiment of the hydrogel of the invention, the reactive end group of the functionalized PEG multi-arm is a thiol, primary amine or an epoxide group, more preferably a thiol group. So, the PEG-Hep hydrogel is formed through a maleimide-thiol covalent reaction between the maleimide of the functionalized heparin and the thiols of the functionalized PEG multi-arm.

Preferably, the hydrogel consists of water and functionalized PEG multi-arm star polymer covalently combined with heparin. More preferably, the hydrogel consists of water and thiol-functionalized PEG multi-arm star polymer covalently combined with maleimide-functionalized heparin. Even more preferably, the hydrogel consists of water and a thiol-functionalized PEG 4-arm star polymer covalently combined with maleimide-functionalized non-fractionated heparin.

Together with the pore size, the expansion of cells inside a biomaterial will strongly depend on the capability of cells to migrate within the material. Therefore, pores should be well connected with each other. A connectivity density of at least 5000 $cm^{-3}$ is preferred; a connectivity above 10000 $cm^{-3}$ or even above 18000 $cm^{-3}$ is preferred for an optimal cell expansion, measured by X-ray microtomography.

The term "connectivity density" used herein, refers to the connectivity number divided by the volume of interest (i.e. the analysed volume of sample) as obtained from the analysis of X-ray microtomography measurements using a Skyscan 1272 high-resolution micro computed tomography (Bruker, USA). Connectivity is a basic concept in mathematics and computer science which equals the minimum number of elements which need to be removed to disconnect the remaining nodes from each other. Connectivity has no units, and it relates to the degree to which parts of an object are multiply connected. Connectivity can be thought of as how many possible ways exist to travel through the pores of the material.

As indicated, the IOPAL hydrogels of the invention have the advantage of possessing improved mechanical and structural properties, such as specific stiffness (storage shear modulus G') and pore size that make the IOPAL hydrogels of the invention very well suited, not only for proliferation of CAR T cells, but also and more important, for CAR expression of T cells, which is considered very valuable for CAR adoptive cell therapies.

Another aspect of the present invention refers to an intermediate product that is obtained when preparing the hydrogel of the present invention. This intermediate product is a composition comprising or consisting of:

a hydrogel having the composition described in the first aspect of the invention (i.e.
hydrogel comprising a functionalized PEG multi-arm star polymer covalently combined with heparin)
and non-crosslinked polymeric spheres, having a diameter between 110 and 150 μm, more preferably 120 μm.

The term "non-crosslinked polymeric spheres" refers to spheres made of a polymer that has not been subjected to any cross-linking, as opposed to crosslinked polymeric spheres, which are widely used in different fields within the materials science field.

In a preferred embodiment of this aspect of the invention, the spheres are PMMA (polymethacrylate) or polystyrene non-crosslinked spheres, more preferably, the spheres are PMMA non-crosslinked spheres.

Another aspect of the invention refers to the method for preparing the hydrogel described in the first aspect of the invention. The method is based in the inverted colloidal crystal technique and comprises the following steps:

i. adding a suspension of non-crosslinked polymeric spheres having a diameter between 110 and 150 μm into a template,
ii. evaporating the solvent comprising the non-crosslinked spheres,
iii. adding an aqueous solution comprising the hydrogel components (i.e: functionalized PEG multi-arm star polymer

and functionalized heparin) into the template and maintain the mixture during a time of at least 24 hours to allow the hydrogel components to react with each other and to jellify, wherein the weight % of functionalized PEG multi-arm in the aqueous solution is between 5 and 8%wt with respect to the weight of the aqueous solution,

iv. dissolving the non-crosslinked polymeric spheres by adding a solution comprising an acid or an organic solvent to the mixture obtained in step iii).

A concentration between 5 and 8%wt of the functionalized PEG multi-arm star polymer in the aqueous solution in step iii. is adequate to obtain the storage shear modulus (G') between 2.5 and 4 kPa. Also, the molecular weight of the functionalized PEG multi-arm star polymer can affect the G'. In a preferred embodiment, the molecular weight (MW) of the functionalized PEG multi-arm star polymer is between 5 and 20 kDa, more preferably, between 7 and 15 KDa, and even more preferably 10 kDa.

The concentration of the functionalized PEG multi-arm star polymer in the aqueous solution in step iii. will be the concentration of functionalized PEG multi-arm star polymer in the hydrogel obtained, assuming no loss of water content.

In a preferred embodiment, the weight % of functionalized PEG multi-arm in the aqueous solution is 6%wt with respect to the weight of the aqueous solution.

The pore size of the final IOPAL hydrogel will depend on the size of these spheres. In a preferred embodiment, the non-crosslinked polymeric spheres have an average diameter of 120 $\mu$m.

In a preferred embodiment of the process of the present invention, the suspension of step i. is an aqueous suspension.

In a preferred embodiment, the spheres are PMMA (polymethacrylate) or polystyrene non-crosslinked spheres, more preferably, the spheres are PMMA non-crosslinked spheres.

In a preferred embodiment, the evaporation step ii. is carried out at a temperature not higher than 40 °C, preferably between 15 and 30 °C, even preferably between 20 and 25 °C.

In a preferred embodiment of the process of the present invention, the mixture is maintained in step iii. at a temperature between 20 and 50 °C, preferably between 30 and 40 °C, even preferably at 37 °C.

In a preferred embodiment of the process of the present invention, the solution of step iv. comprises an acid, preferably a carboxylic acid, more preferably acetic acid.

In a preferred embodiment, the duration of step iv. is between 1 and 5 days, preferably between 2 and 4 days, more preferably about 3 days.

In a preferred embodiment of the process of the present invention, no annealing step is used. Usually, an annealing step (mainly thermal annealing) is carried out in the processes of the state of the art after step i. (between i and ii) in order to fuse together the polymeric particles. However, in the present invention, no annealing step is used. This fact provides the advantage of simplifying the process by avoiding one step and the requirement of having an oven. Moreover, progressive and slow evaporation of the solvent drives particles together to create an ordered and closed-packed array that results in homogenous well-connected pores.

In another aspect, the present invention relates to a hydrogel in the form of an inverse opal scaffold obtained by the process described above.

Then, another aspect of the invention is the hydrogel of the invention or the composition of the invention for use in immunotherapy, preferably in cancer treatments, autoimmune diseases and/or infections.

The term "treating" (or "treat" or "treatment") used herein refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders associated with immune diseases, cancer or infections.

The terms "immunotherapy" used herein, refer to the set of treatment strategies to stimulate, inhibit, or replenish the immune system against cancer, infections, or other diseases as well as to lessen the side effects of very aggressive treatments used against cancer.

Another preferred embodiment is the hydrogel of the invention or the composition of the invention for use in CAR T adoptive cell immunotherapy.

The term "CAR T adoptive cell immunotherapy" refer to a therapeutic approach which involves the isolation and reinfusion or transfer of T lymphocyte immune cells genetically modified to express a CAR receptor into patients to treat disease, preferably to treat cancer, autoimmune disease, or infections. The cells may have originated from the patient or from another individual. In autologous cancer immunotherapy, cells are extracted from the patient, cultured in vitro, transduced with the CAR and returned to the same patient. Comparatively, allogeneic therapies involve cells isolated and expanded from a donor separate from the patient receiving the cells. In the manufacturing of CAR T cell products, it is necessary to achieve a sufficient expansion of the cell population, but also ensure that as many cells as possible are expressing the CAR receptor, since only those cells are able to recognize the target cells.

Examples of types of cancer suitable for immunotherapy include, without limitation, melanoma, colorectal carcinoma, cervical cancer, lymphoma, leukaemia, bile duct cancer, neuroblastoma, lung cancer, breast cancer, or

sarcoma.

Thus, another aspect of the invention refers to the use of the hydrogel of the invention for *in vitro* CART T cell proliferation and /or expression.

The term "CART T cell proliferation" used herein refers to the cell division and cell growth of the cells from the original population to increase the number of CART T cells.

The term "CAR T cell expression" used herein refers to the presentation of CAR receptors in the membrane of T cells. In this regard, when it is stated that an enhancement of the CAR expression occurs, it refers to an increase of the percentage of T cells presenting CAR receptors in their membrane.

In summary, the IOPAL hydrogel described in the present invention provides an improved performance with respect to the hydrogels of the state of the art. Some of the advantages of the hydrogel of the present invention are indicated below:

In the disclosures of the state-of-the-art (Pérez del Rio, E. et al. Biomaterials 259, 120313 (2020) and Santos, F. et al. Biomater. Sci. 10, 3730-3738 (2022)), hydrogels were used to expand T cells, without modifying them to express a CAR. In that case, the hydrogels design focused in the improvement of cell proliferation. The second of those disclosures shows that IOPAL hydrogels, with 3wt% PEG (G' of 0.5 kPa) and 80 μm pore size, were more suitable for T cell expansion than Bulk 3wt% hydrogels.

The hydrogels of this invention, however, were unprecedently studied for the expansion of CAR T cells, which are the clinically relevant cells for CAR adoptive cell therapies. In this case, proliferation is important, but also key is to achieve a cell population that is effective in targeting the tumor cells, i.e. it is equipped with the CAR receptor. Therefore, the CAR expression of T cells is very valuable.

The IOPAL hydrogels of the invention proved to be surprisingly suitable for CAR T cell expansion. The most surprising effect was an enhancement of CAR expression when IOPAL hydrogels of G' of 2.5 to 4 kPa were used. Also proliferation of CAR T cells was enhanced with the hydrogels of the invention compared to those of the prior art.

A complete study of the hydrogels of the inventions and those of the prior art showed that the surprising enhancement of CAR expression is mainly influenced by the hydrogel stiffness, i.e. G', combined with the IOPAL structure. As an example, soft hydrogels like those of the prior art show a significantly worse CAR expression and CAR T cell proliferation compared with the hydrogels of the invention.

Furthermore, a pore size of 110-150 μm was found to be advantageous compared to smaller pore sizes in terms of CAR expression and CAR T cell proliferation.

In a similar way, the presence of heparin in the hydrogels was also seen to be important when the hydrogels of the invention were compared with non-heparin hydrogels.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

## DESCRIPTION OF THE DRAWINGS

**Fig. 1: Hydrogels engineered for the culture of CAR T cells,** a) Synthesis of PEG-Hep hydrogels by thiol-maleimide chemistry, b) Scheme showing the various hydrogel formulations tested and their nomenclature.

**Fig. 2: Physicochemical characteristics of hydrogels engineered for the culture of CAR T cells.** a) ESEM and optical microscopy images showing the porosity of IOPAL hydrogels and the presence of cell clusters inside the pores. b) Graph bar showing the storage modulus (G') of the synthesized hydrogels. c) Summary table of the physicochemical properties of the hydrogels studied for CAR T cell culture.

**Fig. 3: CD4+ CAR T cell culture in suspension, bulk and IOPAL hydrogels.** The proliferation of CD4+ CAR T cells was characterized by flow cytometry and the data was normalized to the suspension culture (C+). a) Proliferation index. b) Expansion index. c) Replication index. CAR expression was also characterized. d) Percentage of CAR+ cells. e) Normalized median fluorescence intensity (MFI) was also analyzed on day 5 by flow cytometry. $N_{donors}$ = 5-6. Each data point is the average of at least 2 replicates. * $p < 0.05$ significance using a Mann-Whitney test.

**Fig. 4.: CAR expression and proliferation profile of CD4+ T cells transduced with different amounts of LV**

**(MOIs of 0.5, 1, 2 and 4) and cultured in suspension or in IOPAL hydrogels.** a) Bar graph shows the percentage of CAR+ cells in each condition. b) Normalized median fluorescence intensity (MFI) of the CAR+ cell population. c) Replication index. $N_{donors} \geq 4$. Each data point is the average of at least 2 replicates. * $p < 0.05$ significance using a Mann-Whitney test.

**Fig. 5: CAR expression and proliferation of CD4+ CAR T cells cultured in different hydrogel formulations.** a) CAR expression profile (percentage of CAR+ cells over total alive cells) of CD4+ T cells transduced with the LV ARI-001 at an MOI of 2 and cultured for 5 days in suspension, PEG3, bulk, IOPAL3.80 and IOPAL6.120 hydrogels ($N_{donors} = 5$). b) Number of copies of the anti-CD19 CAR gene evaluated by RT-qPCR. c) CAR expression of CD4+ CAR T cells cultured in IOPAL hydrogels with different stiffness and pore size ($N_{donors} = 2$). d) CAR expression evaluated in Bulk PEG-Hep and PEG-PEG hydrogels with different stiffness ($N_{donors} = 2$). e) Scheme of the different physicochemical properties of the tested hydrogels and their relative position in their ability to promote CAR expression, from less to more % of CAR+ cells. f) Replication index ($N_{donors} = 5\text{-}6$). Each data point is the average of at least 2 replicates. * $p < 0.05$ significance using a Mann-Whitney test.

**Fig. 6.** (a) Electrostatic potential from Poisson-Boltzmann (PB) calculations evaluated over the surface of the VSV-G protein (in kT/e = 25 mV units). (b) Binding of heparin as identified by docking calculations (configuration with the largest number of contacts between the protein and heparin). The capture shows how heparin (very electronegative) binds a positively charged pocket of the protein. (c) Snapshot from Molecular Dynamics (MD) simulations showing an equilibrated configuration obtained from the structure in (b). The zoom shows the aminoacids in contact with heparin (note the rotation of the structure in order to show the view from inside the protein). Image created with Visual Molecular Dynamics (VMD).

## EXAMPLES

## MATERIALS AND METHODS

### 2.1 Materials

Heparin was acquired from Acros (Fisher Scientific, USA) and functionalized with maleimide as previously described. 4-arm thiol-terminated poly(ethylene oxide) (Mn 10000 g/mol) was purchased from Nanosoft Polymers (USA). 10% w/v aqueous suspension of poly(methyl methacrylate) (PMMA) beads with diameters of 78.3 ± 1.7 μm and 119.3 ± 2.1 μm were purchased from microParticles GmbH (Germany). Fetal bovine serum (FBS), penicillin/streptomycin (P/S), CellTrace carboxyfluorescein diacetate succinimidyl ester (CFSE) cell proliferation kit, Dynabeads, streptavidin-allophycocyanin (APC) conjugate and Hoechst were provided by Thermo Fisher Scientific (USA). Lymphoprep was obtained from Stemcell Technologies (Canada). The CD4+ T cell isolation kit was acquired from Miltenyi Biotec GmbH (Germany). The anti-human CD3 FITC, CD4 PE, CD45RO FITC antibodies and their controls used for flow cytometry were acquired from Immunotools GmbH (Germany), whereas CD62L PE and its control were bought from BioLegend (USA). Anti-HIV1 p24 goat antibody and anti-goat H&L Alexa Fluor 488 antibodies were acquired from Abcam (UK). For the real-time quantitative PCR (RT-qPCR), LightCycler 480 SYBR Green I Master and 384-Multiwell plate Cat. No. 04707516001 were purchased from Roche (Germany). Sterile nuclease-free H2O AM9906 was obtained from Thermofisher (USA), Enzyme restriction NotI-HF. Cat. No. R0189S from New England Biolabs (USA), and QIAquick Gel Extraction Kit and DNeasy Blood & Tissue Kit (50) (Ref 69504) were from Qiagen (Germany). N-(2-aminoethyl)maleimide trifluoroacetate salt (AEM), 1-hydroxybenzotriazole hydrate (HOBT), N-(3-dimethylamino-propyl)-N-ethylcarbodiimide hydrochloride (EDC·HCl), 2-(N-morpholino)ethanesulfonic acid (MES), Dulbecco's phosphate buffered saline (PBS), Roswell Park Memorial Institute (RPMI)-1640 cell culture media, and the rest of the products not otherwise specified were purchased from Merck (USA).

### 2.2. Hydrogel preparation

Bulk and IOPAL PEG-Hep hydrogels were prepared following previously reported protocols (Pérez del Río, E. et al. Biomaterials 259, 120313 (2020);
Santos, F. et al. Biomater. Sci. 10, 3730-3738 (2022). The starting components used were heparin-maleimide (Hep-Mal) (Baldwin, A. D. & Kiick, K. L. Polym. Chem. 4, 133-143 (2013)) and commercial 10 kDa 4-arm PEG-SH (PEG). PEG-Hep hydrogels were formed through a Michael reaction between Hep-Mal and PEG in a molar ratio of 1.5:1 in PBS or cell media. The hydrogels were prepared in 5 mm-diameter wells of a homemade Teflon template and had a volume of 30 μL. For bulk hydrogels, the mixture was directly added to the wells and incubated at 37 °C at least overnight resulting in a covalently bound hydrogel. For IOPAL hydrogel formation, 100 μL of a 10% w/v aqueous suspension of non-crosslinked polymethyl methacrylate (PMMA) beads (78.7 ± 1.7 μm or 119.3 ± 2.1 μm diameter) was added to the same 5 mm wells and left for at

least 24 h or until the solvent evaporated. The opal was then formed, and the mixture of components was added on top of the PMMA opal and left to infiltrate. Incubation was held at 37 °C for at least 2 days. Then, the hydrogel containing the opal was removed from the template and introduced in glacial acetic acid for at least 48 h (40 °C, 150 rpm; orbital shaker) to dissolve the PMMA beads of the opal. Finally, the IOPAL hydrogels were washed 3 times in PBS to remove the acid.

PEG-PEG hydrogels were prepared in the same Teflon template by mixing 2 kDa Mal-PEG-Mal and 2 kDa 4-arm PEG-SH at a 2:1 molar ratio. The mixture creates a hydrogel in a few minutes.

**2.3 Hydrogel characterization**

2.3.1 Environmental Scanning Electron Microscopy (ESEM)

A FEI Quanta 650F Environmental scanning electron microscope (Thermo Fisher Scientific, USA) was used to image hydrated hydrogels. While SEM is usually thought for dry conductive samples, in this case a protocol was optimized for the imaging of hydrated hydrogels. Basically, a temperature of 5 °C was used, and the pressure was abruptly dropped from 900 to 100 or 200 Pa in order to evaporate the water from the pores and exposing the hydrogel structure.

2.3.2 Optical Microscopy

Optical microscopy captures were taken using an inverted microscope IM-3 (Optika Microscopes, Italy) and an attached Optikam PRO6 Digital Camera (optika Microscopes, Italy).

2.3.3 Rheology

Measuring the storage shear modulus (G') is a common way to characterize hydrogel stiffness. Using a small-amplitude oscillatory shear (SAOS) technique, the storage shear modulus (G') can be obtained from the linear plateau observed at low frequencies of frequency sweeps (Zuidema, J. M., et al.J. Biomed. Mater. Res. Part B 102, 1063-1073 (2014). https://doi.org/doi:10.1002/jbm.b.33088).Strain sweeps were performed to determine the linear-viscoelastic region of the hydrogels and choose and appropriate strain for the subsequent frequency sweeps. The experiments were performed at 37 °C. The equipment used was a Rheometer HAAKE RheoStress RS600 (Thermo Electron Corporation, USA) with a 10 mm diameter rotor.

**2.4. Lentivirus preparation**

The anti-CD19 CAR cloning and lentivirus production was performed as previously described (Castella, M. et al. Molecular Therapy Methods & Clinical Development 12, 134-144 (2019)). The CAR sequence comprises a variable light and heavy regions extracted from A3B1 hybridoma cells, signal peptide, A3B1 scFv, CD8 hinge, and transmembrane regions 4-IBB and CD3z. The complete CAR sequence was cloned into a third-generation lentiviral vector. Lentiviral particles were produced by transfecting HEK293T cells with the transfer vector, packaging plasmids and envelope plasmid using Polyethylenimine (PEI) with molecular weight of 25 kDa. $6 \cdot 10^6$ HEK293T cells were plated 24 h before transfection in 10-cm dished. Then, 14 µg total DNA was diluted in serum-free DMEM and, subsequently, 35 µg PE! were added and incubated for 20 min at RT. The mix was added to the cells cultured in 7 mL DMEM complemented with 10% FBS, and the media was replaced after 4 h of incubation. Viral supernatants were collected after 48 h and clarified by centrifugation followed by filtration trough a 0.45 µm filter. Ultracentrifugation at 26000 rpm for 2 h and 30 min was used to concentrate the viral particles before resuspending them in XVivo15 media and storing them at -80°C until use.

2.4.1 Lentivirus Titration

Lentivirus titration was done following a previously described protocol (Castella, M. et al. Molecular Therapy Methods & Clinical Development 12, 134-144 (2019)). In brief, the number of transducing units per volume unit (TU/mL) was determined by the limiting dilution method. In summary, serial 1:10 dilutions of the viral particles were prepared and added on top of HEK293T cells in complete DMEM with 5 µg/mL Polybrene. The cells were trypsinyzed 48 h later and stained with an APC-conjugated anti-mouse immunoglobulin G antibody. The result was analyzed by flow cytometry, and a dilution corresponding to 2-20 % of positive cells was used to calculate the viral titer.

**2.5. CAR T cell culture**

Buffy coats from healthy adult donors were supplied by "Banc de Sang i Teixits" (Barcelona, Spain). Permission for using such samples was obtained after the approval of the research project by the "Ethics Committee on Animal and

Human Experimentation" of the Autonomous University of Barcelona (No. 5099). An established protocol based on a density gradient centrifugation and a magnetic selection was followed to obtain primary human CD4+ T cells (Pérez del Rio, Biomaterials 259, 120313 (2020);Santos, F. et al. Biomater. Sci. 10, 3730-3738 (2022)). Only samples that were >90% (usually >95%) CD3 and CD4 positive were employed. After isolation, CD4+ T cells were seeded on 96-well plates, either in suspension or on top of a bulk or IOPAL hydrogel. The cells were seeded at a concentration of $10^6$ cells/mL ($10^5$ cells per well) and a 1:1 ratio of Dynabeads (1 bead per cell) in supplemented RPMI cell media. The cell culture was kept in the incubator at 37 °C and 5% $CO_2$.

One day after the CD4+ T cell seeding, cells were transduced with a LV viral vector. First, the amount of LV to add per well was calculated according to the LV batch titer and the desired MOI, following (1). The calculated amount of LV was added to each well diluted in RPMI supplemented cell media to achieve a volume per well of 10 µL.

$$\mu L\ LV\ to\ add = \frac{nr\ T\ cells \cdot MOI}{titer} \cdot 1000 \quad (1)$$

Two days after the cell seeding, 100 µL of RPMI supplemented cell medium were added to each well. On day 5, cells were collected from the wells. Whenever cells were cultured in hydrogels, the hydrogels were mechanically smashed and passed through a 70 µm cell strainer to maximize cell recovery. Otherwise, vigorous pipetting was used to harvest the cells. Once collected, cells were separated from Dynabeads using a magnet. Optionally, CAR T cells culture was continued by counting them every day and re-adjusting their concentration at 1 M/mL on day 5 and at 0.7-0.8 M/mL from day 6 on-wards.

### 2.6. CAR T cell proliferation and CAR expression

For proliferation analysis, CAR T cells were stained with a CFSE cell proliferation kit following the instructions of the manufacturer before seeding. On day 5 or 6, debeaded cells were washed and analyzed by flow cytometry. To minimize the variability caused by the different donors, the results obtained were normalized to the positive control of each donor, which was assigned a value of 1. For CAR expression analysis, CAR T cells were washed, incubated first with a biotinylated anti-CAR antibody and then with streptavidin-APC (Peinelt, A. et al. Front. Immunol. 13, 830773 (2022)). A non-transduced condition was used as a control, which was used to establish the gating for CAR+ cells of the cytometry results obtained. Proliferation and CAR expression analysis could be performed simultaneously if needed.

### 2.7. Flow cytometry

Flow cytometry experiments were performed with a CytoFLEX LX (Beckman Coulter, USA) and a BD FACSCanto (BD Bioscience, USA). For all measurements, cells were washed and resuspended in PBS with 0.1% FBS. The FlowJo software was used to analyze the data.

### 2.8. Confocal microscopy

Cells were cultured on confocal 96-well plates (black walls and glass bottom) and fixed on day 3 by incubating for 20 min at RT with 4% paraformaldehyde (PFA). For cell permeabilization, the fixed cells were then treated with Triton X-100 0.1% for 5 min. Then, nonspecific protein binding was blocked by incubating with 1% BSA/0.1% Triton X-100 in PBS for 30 min-1h at room temperature. Subsequent antibody incubations were performed using this same blocking buffer. The antibody incubations consisted of, first, an anti-p24 goat antibody at a 1:200 dilution overnight at 4 °C; and second, an anti-goat H&L and Hoechst at dilutions 1:200 and 1:1000 for 1h at room temperature. The samples were subsequently observed in an Olympus microscope at 63 X.

### 2.9. Real-time quantitative PCR (qPCR)

For gene quantification, CD4+ CAR T cells were expanded until day 5 as described in previous section 2.5, and the cells were collected, debeaded, and washed in PBS. Then, dry pellets were stored at -80 °C.

For the qPCR experiment, a LightCycler 480 Instrument was used. All samples and standards were run in duplicates, and with a negative control containing $H_2O$ instead of sample, also in duplicates.

For each one of the tested plasmids, a standard curve was prepared with n° of copies per µL of: $10^8$, $10^7$, $10^6$, $10^5$, $10^4$, $10^3$, $10^2$. To prepare the standard, 5 µg of plasmid were digested overnight at 37 °C with the NotI restriction enzyme. The resulting product was run in an agarose gel and purified using the QIAquick Gel Extraction Kit. DNA concentration was tested using a NanoDrop spectrophotometer and adjusted for the preparation of the standard curve.

The DNA of the samples to be analyzed was extracted using the DNeasy Blood & Tissue Kit (Qiagen). The DNA

concentration was tested using a NanoDrop spectrophotometer and adjusted to 100 ng/mL when possible, or otherwise at 25 ng/mL. Two genes (in duplicates) were tested for each sample. The GATA2 gene was used to normalize the results. The WPRE gene was used to quantify the ARI-001 anti-CD19 CAR presence. In each well of a 384-wellplate, we added 3 μL $H_2O$, 5 μL of LightCycler 480 SYBR Green I Master (2X), 0.5 μL of primer F, 0.5 μL of primer Rand 1 μL of DNA sample. The wellplate was sealed, centrifuged and kept in the dark until the experiment was run.

The thermocycler protocol consisted on a 95 °C 5 min first step followed by 40 cycles of: 95°C for 10 seconds, 58 °C for 10 seconds and 72 °C for 5 seconds. Followed by 5" at 95°C, 1 minute at 65 °C and then 97°C.

The standard curve was analyzed, and the most diluted point eliminated whenever linearity was not maintained. The analysis of the melting curve was performed to make sure a single peak corresponding to a single amplicon was obtained for each sample. Finally, the data was analyzed to obtain the number of copies per genome of the WPRE gene, which equal the number of copies of the CAR in each genome. To achieve this, the samples number of copies were obtained by interpolating the results in the standard curves. Then, the GATA2 gene, which has 2 copies per genome, was used to normalize.

$$\frac{copies\ GATA2}{2} = n^{\underline{o}}\ of\ genomes \qquad (2)$$

$$\frac{copies\ CAR}{genome} = \frac{copies\ WPRE}{genome} = \frac{2 \cdot copies\ WPRE}{copies\ GATA2} \qquad (3)$$

### 2.10. Computer Simulations

We have performed electrostatic potential and docking calculations and Molecular Dynamics (MD) simulations of a VSV-G lentiviral protein and heparin. Preparation and analysis of structures and trajectories was done with VMD (Humphrey, W., J. Mol. Graph. 14, 33-38 (1996)) and Chimera (Pettersen, E. F. et al. J. Comput. Chem. 25, 1605-1612 (2004)) softwares.

The VSV-G lentiviral protein structure used in all the calculations was obtained from the Protein Data Bank database (code PDB: 5I2S) and it was protonated at pH=7 using the PDB Reader & Manipulator of CHARMM-GUI. Electrostatic potential calculations were made at the nonlinear Poisson-Boltzmann (PB) level of theory using the APBS 3.4.1 software. Docking simulations between heparin and a VSV-G protein were performed using ClusPro 2 software. We considered a standard heparin fragment, a tetrasaccharide. The configurations predicted by docking were further refined by using them as initial configurations in MD performed with the NAMD 2.14 software. All simulations were done at T=298 K in explicit water and RPMI buffer. The force field employed in the simulations is CHARMM36, which includes an appropriate parametrization for the protein and the heparin molecule. Our GitHub repository also provides open access to data from calculations, including structure files and sample scripts.

## 3. RESULTS AND DISCUSSION

### 3.1. Synthesis of hydrogels

PEG-Hep hydrogels in its unstructured (Bulk) or IOPAL form have been shown to improve the expansion of primary human CD4+ T cells by providing adequate mechanical and biochemical stimuli to the cells. Here, a new generation of PEG-Hep hydrogels was designed, synthesized, and characterized to dissect the impact of key physicochemical parameters such as chemical composition, stiffness, and structure (pore size and interconnectivity) on the culture of CD4+ CAR T cells (Fig. 1).

PEG-Hep hydrogels were prepared using 4-arm-PEG-SH and maleimide-functionalized unfractioned heparin (Fig. 1a). First, new IOPAL hydrogels with a pore size 120 μm were prepared (IOPAL3.120), enlarging the previously described hydrogels with a pore size of 80 μm (IOPAL3.80). Then, another two new types of IOPAL hydrogels with a higher weight concentration, 6wt% instead of the previously used 3wt%, were also created (IOPAL6.80 and IOPAL6.120) in order to provide hydrogels with two different stiffnesses The resulting G' for all hydrogels is listed in Figure 2c. In the next step, we also added our Bulk hydrogels with the two weight concentrations, 3 (Bulk3) and 6wt% (Bulk6). Finally, novel PEG-PEG hydrogels were prepared from 4-arm-PEG-SH and PEG-bis-maleimide, i.e., hydrogels that do not comprise heparin, in the two concentration forms (PEG3 and PEG6). By preparing all the mentioned combinations, we analyzed a total of 8 different types of hydrogels (Fig. 1b).

The hydrogels prepared in this work were characterized by different techniques such as rheology, optical microscopy and ESEM. A table summarizing the physicochemical properties of the hydrogels and the nomenclature used can be found in Fig. 2c.

### 3.2. Characterization of hydrogels

First, the pore structure of hydrogels was explored by ESEM (Fig. 2a). With this technique, we confirmed that the IOPAL hydrogels have a narrow pore size distribution with pores of sizes around that of the porogens used, which are of 80 and 120 $\mu$m, in this case. Bulk hydrogels, unlike IOPALs, have only the intrinsic material porosity, which show pores of around 55 $\mu$m for Bulk3 and 20 $\mu$m for Bulk6. Finally, PEG hydrogels show a more irregular microstructure, with parts containing no visible pores combined with others of irregular porosity. It is also worth mentioning that the pores of IOPAL hydrogels can already be visualized with an optical microscope. During cell culture, clusters of CD4+ CAR T cells form inside the pores of the hydrogels (Fig. 2a). This compartmentalization reminds that of lymph nodes, in which T and B cells are separated in different zones, and cells cluster around dendritic cells. In the next step, the mechanical properties of the prepared hydrogels were determined by small-amplitude oscillatory shear (SAOS) rheology measurements at 37 °C. In particular, the frequency sweeps were performed at a constant strain of 10 Pa and frequencies ranging from 0.1 to 15 Hz (Fig. 2b). These conditions ensured that all hydrogels were tested within their linear viscoelastic regime.

PEG-Hep hydrogels with 3wt% PEG showed storage moduli (G') of 1.5 $\pm$ 0.1 kPa for Bulk3, 0.5 $\pm$ 0.1 kPa for IOPAL3.80, and 0.7 $\pm$ 0.2 kPa for IOPAL3.120. The difference between the Bulk and IOPAL hydrogels may be accounted by the fact that IOPAL hydrogels have better interconnected pore structures (and larger pore sizes). On the other hand, PEG-Hep hydrogels with 6wt% PEG showed G' of 3.5 $\pm$ 0.7 kPa for Bulk6, 2.6 $\pm$ 0.9 for IOPAL6.80, and 3.1 $\pm$ 0.8 kPa for IOPAL6.120. As expected, G' was quite lower when hydrogels were prepared with a 3wt% PEG compared to those prepared with a 6wt%. Finally, PEG3 and PEG6 hydrogels, which were prepared only with PEG reactants of short chains (2kDa vs > 10kDa), presented higher stiffnesses. In particular, they presented G' of 2.0 $\pm$ 0.6 kPa for PEG3 and 8.8 $\pm$ 1.6 kPa for PEG6.

### 3.3. Primary human CD4+ CAR T expansion and CAR expression

Primary human CD4+ CAR T cells stained with CFSE were expanded in our basic hydrogels, Bulk3 and IOPAL3.80 hydrogels, as well as in suspension with Dynabeads. These hydrogels were chosen as they have already demonstrated their capacity to improve CD4+ T-cell culture of non-transduced cells. At day 5, cells were collected and the proliferation indexes were assessed by flow cytometry. Cell expansion was continued in suspension until day 9, in which CAR expression was also assessed by flow cytometry.

A significant enhancement of the proliferation indexes was found when cells were seeded on both Bulk3 and IOPAL3.80 hydrogels compared to cells seeded in suspension (Fig. 3a-c). Specifically, cells cultured in Bulk3 and IOPAL3.80 hydrogels showed replication indexes 60% and 50% higher than those of cells expanded in suspension, in line with the previously reported results for non-transduced T cells. However, a subtle difference was observed between transduced and non-transduced cells expanded in suspension (10% higher replication index for non-transduced cells), which may account for the tonic signaling produced by CARs. In summary, we could confirm the preference of cells for having a 3D microenvironment in which they can interact with their extracellular matrix compared to suspension.

To assess CAR expression, the percentage of cells showing positive expression of the CAR (Fig. 3d) and the median fluorescence intensity (MFI; Fig. 3e), which gives a qualitative measurement of the level of CAR expression in each cell, were analyzed. In this case, cells expanded in IOPAL3.80 hydrogels showed the highest percentage of CAR+ cells (around 67%), while cells expanded in suspension or in Bulk3 hydrogels showed a similar percentage of CAR+ cells of around 55%. No differences between conditions were detected in the MFI. The significant enhancement of the CAR expression in terms of the percentage of CAR+ cells observed in IOPAL3.80 hydrogels triggered a further investigation of the impact of the hydrogel characteristics.

### 3.4. Effect of MOI on CD4+ CAR T proliferation and CAR expression

The MOI used in culturing CAR T cells has an impact on the characteristics of the resulting CAR T cellular product. The optimal value is a tradeoff between high values prompt to tonic signaling and low values that could result in poor CAR expression. To examine this, CD4+ CAR T cells transduced with MOIs of 0.5, 1, 2 and 4 were cultured in both suspension and IOPAL3.80 conditions (Fig. 4a).

CAR expression analysis showed how increasing MOI results in a proportional increase of the % of CAR+ cells, as expected. For cells in suspension (C+), MOIs of 0.5, 1, 2 and 4 resulted in CAR+ % of 31, 42, 53, and 70, while percentages of 39, 53, 66, and 77 were obtained for cells cultured in IOPAL3.80 hydrogels. The difference is statistically significant in the case of MOI 2 (53% in suspension versus 66% in IOPAL3.80). Moreover, it is relevant to notice that the same CAR+ % was achieved using an MOI of 2 in suspension and an MOI of 1 in IOPAL3.80 hydrogels.

Cells cultured in IOPAL3.80 hydrogels not only show a higher CAR+ % for the same MOI, but also have a slight tendency to show a higher MFI (Fig. 4b). In this case, however, there is no statistical difference between IOPAL3.80 and suspension conditions.

The results obtained with the different MOI are in line with previous studies,[38,70] in which the transduction of the CAR depends on two main factors. First, the MOI, which determines the number of vector-cell encounters following a Poisson distribution. Second, the efficiency of transduction, which determines how many of those encounters end in transduction of the CAR, and it will depend on the cell type/lentiviral vector. However, the addition of a hydrogel might disturb the Poisson distribution by making cell-lentiviral vector encounters more probable.

CFSE-stained cells were analyzed by flow cytometry on day 5 (Fig. 4c and S5). As mentioned in the methods, to reduce the intrinsic donor-to-donor variability in the experiments due to the primary origin of cells, results were normalized to the non-transduced cells cultured in suspension (C+ NT). The results seem to show a slight decrease of the indexes as the MOI increases, especially for MOIs of 2 and 4, which is probably caused by tonic signaling. Also, cells cultured in IOPAL3.80 show higher proliferation, expansion, and replication indexes compared to cells cultured in suspension with the same MOI (enhancement of up to 20%). However, the differences were not found to be statistically significant. The improvement of the proliferation, expansion, and replication indexes when culturing non-transduced CD4+ T cells in IOPAL3.80 hydrogels had already been observed as mentioned before.[31] Here, we confirmed that transduced cells with different MOIs show a similar behavior.

### 3.5. Impact of the physicochemical properties of hydrogel formulations on CAR expression and proliferation

Next, we cultured CD4+ CAR T cells transduced with an MOI of 2 in hydrogels with different physicochemical parameters, with the aim of elucidating the impact of each parameter on CAR expression and proliferation (Fig. 5).

Fig. 5a shows the percentage of CAR+ cells found after 5 days of culture in four different hydrogels, the previously described Bulk3 and IOPAL3.80 hydrogels, as well as the PEG3 hydrogels to assess the effect of the presence/absence of heparin, and the IOPAL6.120 which features a more extreme architecture with larger pores and stiffer characteristics. The percentage of CAR+ cells in suspension (C+) was found to be slightly over 50%, while in the non-heparin-containing PEG3 hydrogels was 55% and in the Bulk3 hydrogels 57%. The IOPAL3.80 showed a small increase with a percentage of CAR+ cells of around 64%. Interestingly, the IOPAL6.120 exhibited the highest (and statistically significant) CAR expression with 75% of CAR+ cells. All hydrogel conditions showed increased CAR expression compared to the C+, pointing to a positive 3D effect of hydrogels compared to the 2D suspension culture, as previously shown.

The reported interaction between heparin and LV, which is explained by the natural viral affinity to heparan sulfate as cell-binding strategy, might also play a role. In this regard, the CAR expression of CD4+ cells cultured in Bulk hydrogels with and without heparin shows a slight increase when heparin is present. A plausible hypothesis is that both cells and LV are drawn towards the heparin-containing hydrogel structure, therefore increasing their potential encounter. The IOPAL structure, which should further increase the interaction between the two components due to its interconnected pores of a defined size, proved to be advantageous for CAR expression compared to homogeneous Bulk hydrogels. However, the highest percentage of CAR+ was obtained with the IOPAL6.120 hydrogel, which not only contains the highest amount of heparin, but also the largest interconnected pores. Moreover, higher amounts of PEG/heparin have been linked to higher stiffness.

The same conditions were analyzed by qPCR to determine the CAR sequence in the cells genome instead of receptor on the cell membranes (Fig 5b). The experiment showed a median number of copies per genome of around 1 in the C+, Bulk3, and PEG3 conditions. In the IOPAL3.80 and IOPAL6.120, however, cells presented a significantly higher number of gene copies, proving that the CAR transduction was more efficient when CD4+ T cells were cultured in IOPAL hydrogels compared to other hydrogels or is suspension.

To shed light on the previous findings, the CAR expression of CD4+ CAR T cells cultured in IOPAL hydrogels, with four possible combinations arising from 3wt% or 6wt% PEG and 80 or 120 $\mu$m porogen sizes, was studied by flow cytometry (Fig 5c). The percentage of CAR+ cells was 65% and 66% in IOPAL3.80 and IOPAL3.120, respectively; and 72% and 74% in IOPAL6.80 and IOPAL6.120, respectively. Thus, the pore size does not seem to be responsible for the differences observed in CAR expression. On the other hand, the matrix stiffness and/or amount of heparin seems to play a key role.

To further investigate this effect, Bulk PEG-Hep and PEG hydrogels also with a 3wt% and 6wt% of PEG were evaluated (Fig 5d). The percentage of CAR+ cells was 40% and 43% in Bulk3 and Bulk6 hydrogels, respectively; and 41% and 38% in PEG3 and PEG6 hydrogels, respectively. Although non-significant results were obtained, there is a slight improvement when moving from 3 wt% to 6wt% in Bulk PEG-Hep hydrogels, which have heparin, unlike when comparing PEG hydrogels, suggesting an effect of the GAG. It is worth noting that PEG hydrogels are stiffer than Bulk PEG-Hep hydrogels with the same proportion of PEG, as their components have shorter chains. In summary, the results seem to point to a synergy between heparin presence, IOPAL architecture, and appropriate stiffness for a positive modulation of the CAR expression of CD4+ CAR T cells (Fig 5e).

Additionally, the expansion of the CD4+ CAR T cells in the different hydrogel formulations was evaluated, as performed before (Fig. 5f). The trend observed was similar to the one of the CAR expression, with a significant improvement when cells were cultured in IOPAL6.120 hydrogels. However, the PEG3 hydrogels showed the second-best condition, significantly improving CAR T proliferation indexes compared to C+ and Bulk3 conditions. The higher stiffness of PEG hydrogels could explain these results, as this could lead to an improved mechanotransduction that would favor activation, migration, and ultimately proliferation of T cells. In this regard, recent studies point to the importance of the forces that T cells can exert through the TCR towards their activation stimuli. The observed trend is that, in the range of physiological stiffness, which is around 0.5-100 kPa, stiffer substrates are preferred by T cells resulting in improved migration and stronger activation. This observed trend is in agreement with PEG3 and IOPAL6.120 hydrogels, which are stiffer than Bulk3 and IOPAL3.80 hydrogels, showing higher CD4+ CAR T proliferation.

Furthermore, there seems to be an influence of the IOPAL structure as well as the heparin content in cell proliferation, as the IOPAL6.120 hydrogels exhibited the highest proliferation. The IOPAL structure is characterized by a narrow pore size distribution and an excellent interconnectivity of the pores. This defined architecture allows a good infiltration and migration of T cells, and provides cavities for cells to cluster (Fig. 2a), with a compartmentalization that may resemble that of lymph nodes.

### 3.6. LV distribution in the hydrogels by confocal microscopy

While LV size and morphology makes it difficult to visualize it by confocal microscopy, we attempted to see its distribution in the hydrogel samples by staining the capsid protein p24. Unfortunately, no unambiguous and specific signal could be obtained.

### 3.7. Molecular basis for the interaction between lentiviral particles and heparin

Further evidence of the interaction between lentiviral particles and heparin was explored using molecular simulations.

More specifically, we have studied the interaction between the VSV-G protein (responsible for the interaction of the lentiviral particles and the environment) and a heparin fragment (a dimer). Our electrostatic calculations (Fig. 6a) show that the VSV-G protein has a region with a large positive electrostatic potential ($\approx$125 mV) suitable for a strong electrostatic interaction with the negatively charged heparin. It should be noted that this region of the protein is the one interacting with the LDL receptor and therefore responsible for the interaction of the virus with cells.

Docking calculations indicate that heparin is able to attach electrostatically to this region, with several possible poses (see Fig. 6b for the pose with the largest number of contacts with the protein for further possible configurations). The stability of the most probable configurations with heparin bound to VSV-G, as identified by docking calculations, was confirmed by MD simulations. We considered the configurations identified by docking as starting configurations for long MD runs

The equilibrated configurations from MD are very close to the ones identified by docking calculations (see Fig. 6c for the refinement of the structure of Fig. 6b for other possible configurations). As seen in Fig. 6c, in this equilibrated configuration the heparin fragment is bound to an average of $\approx$12 protein aminoacids, corresponding to 6 aminoacids per heparin monomer. The interaction is essentially electrostatic but also some hydrogen bonds are present (3.5 on average). In summary, our calculations confirm the expectation that the region with high positive electrostatic potential of the VSV-G protein is able to bind heparin. Therefore, theoretical calculations support the concept that the lentiviral particles bind electrostatically the hydrogels containing heparin.

## 4. CONCLUSIONS

We have shown that the manufacture of primary human CD4+ CAR T cells in engineered PEG-Hep hydrogels can positively modulate their proliferation and efficiency of CAR transduction, due to the presence of a 3D support, its microstructure, the stiffness of the matrix, and the presence of heparin.

The findings of this study shed some light on the fundamental understanding of the transduction step in CAR T fabrication, and point to a way to improve such a process, which would be a great step towards a more extended clinical implementation of CAR T therapy.

## Claims

1. Hydrogel, comprising:
a functionalized PEG multi-arm star polymer covalently combined with heparin, wherein the hydrogel is in the form of an inverse opal scaffold and has a storage shear modulus (G') between 2.5 and 4 kPa, as measured by small-amplitude oscillatory shear (SAOS) rheology.

2. The hydrogel, according to claim 1, that has a storage shear modulus (G') between 2.6 and 3.5 kPa, more preferably between 3.0 and 3.5 kPa and even more preferably, 3.1 kPa.

3. The hydrogel, according to claim 1 or 2, that has an average pore diameter between 110 and 150 μm, more preferably 120 μm, as measured by environmental scanning electron microscopy (ESEM).

4. The hydrogel, according to any of the preceding claims, wherein PEG is a thiol-functionalized PEG multi-arm star polymer.

5. The hydrogel, according to any of the preceding claims, wherein heparin is non-fractionated heparin.

6. The hydrogel, according to any of the preceding claims, wherein heparin is maleimide-functionalized heparin.

7. The hydrogel, according to any of the preceding claims, wherein the functionalized PEG multi-arm star polymer is between 5%wt to 8%wt on the total weight percentage of hydrogel, more preferably between 6%wt to 8%wt.

8. The hydrogel, according to any of the preceding claims, wherein the functionalized PEG multi-arm star polymer has a molecular weight (MW) between 5 and 20 kDa, more preferably, between 7 and 15 KDa.

9. A composition for the preparation of the hydrogel of any of the preceding claims comprising:

   a hydrogel comprising a functionalized PEG multi-arm star polymer covalently combined with heparin and non-crosslinked polymeric spheres having a diameter between 110 and 150 μm,

10. A process for the preparation of the hydrogel in the form of an inverted opal scaffold described in any of the claims 1 to 9, comprising the following steps:

   i. adding a solution of non-crosslinked polymeric spheres having a diameter between 110 and 150 μm into a template,
   ii. evaporating the solvent comprising the non-crosslinked spheres,
   iii. adding an aqueous solution comprising a functionalized PEG multi-arm star polymer and heparin into the template and maintaining the mixture during a time of at least 24 hours to allow the hydrogel components to react with each other and jellify, wherein the weight % of functionalized PEG multi-arm in the aqueous solution is between 5 and 8%wt with respect to the weight of the aqueous solution,
   iv. dissolving the non-crosslinked polymeric spheres by adding a solution comprising an acid or an organic solvent to the mixture obtained in step iii).

11. The process, according to the preceding claim 10, wherein the solution of step iv. comprises acetic acid.

12. The process, according to any of the preceding claims 10 to 11, wherein the duration of step iv. is between 1 and 5 days.

13. The hydrogel of any of the claims 1 to 8 for use in immunotherapy.

14. The hydrogel for use, according to claim 13, for use in CAR T adoptive cell immunotherapy.

15. Use of the hydrogel of any of the claims 1 to 8 for *in vitro* human car t cell expression.

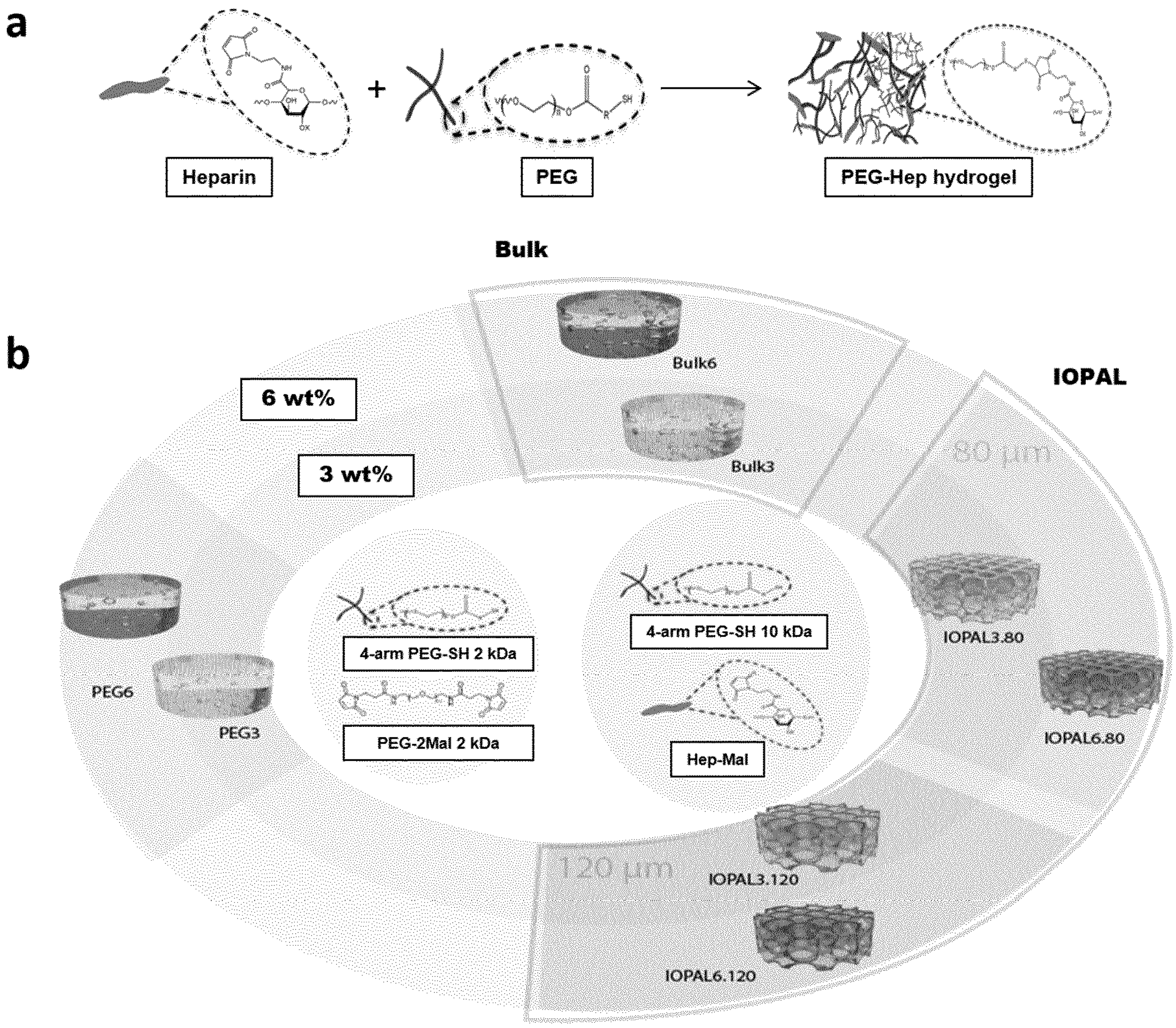
a
Heparin
PEG
PEG-Hep hydrogel
Bulk
b
Bulk6
Bulk3
IOPAL
6 wt%
3 wt%
80 µm
4-arm PEG-SH 2 kDa
PEG-2Mal 2 kDa
4-arm PEG-SH 10 kDa
Hep-Mal
IOPAL3.80
IOPAL6.80
PEG6
PEG3
120 µm
IOPAL3.120
IOPAL6.120

FIG. 1

a

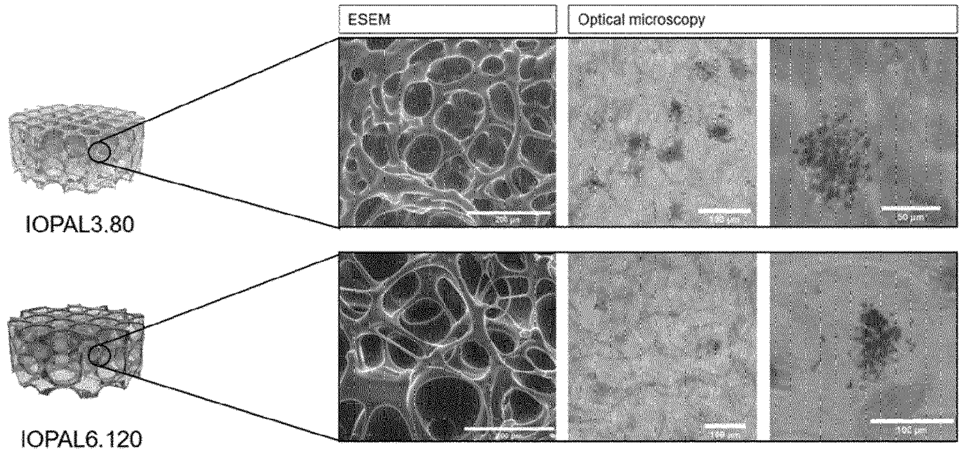


b

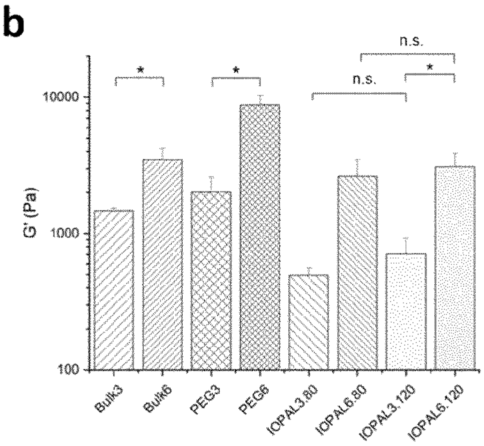


c

| Hydrogel nomenclature | Components | PEG wt% | Architecture | Approx average pore size | Stiffness (Ge) |
|---|---|---|---|---|---|
| PEG3 | 4arm-PEG-SH and PEG-bisMal | 3 wt% | Bulk | irregular | 2,0 kPa |
| PEG6 | | 6 wt% | | irregular | 8,8 kPa |
| Bulk3 | 4arm-PEG-SH and Hep-Mal | 3 wt% | | 55 µm | 1,5 kPa |
| Bulk6 | | 6 wt% | | n.m. | 3,5 kPa |
| IOPAL3.80 | | 3 wt% | IOPAL | 80 µm | 0,5 kPa |
| IOPAL6.80 | | 6 wt% | | 80 µm | 2,6 kPa |
| IOPAL3.120 | | 3 wt% | | 120 µm | 0,7 kPa |
| IOPAL6.120 | | 6 wt% | | 120 µm | 3,1 kPa |

FIG. 2

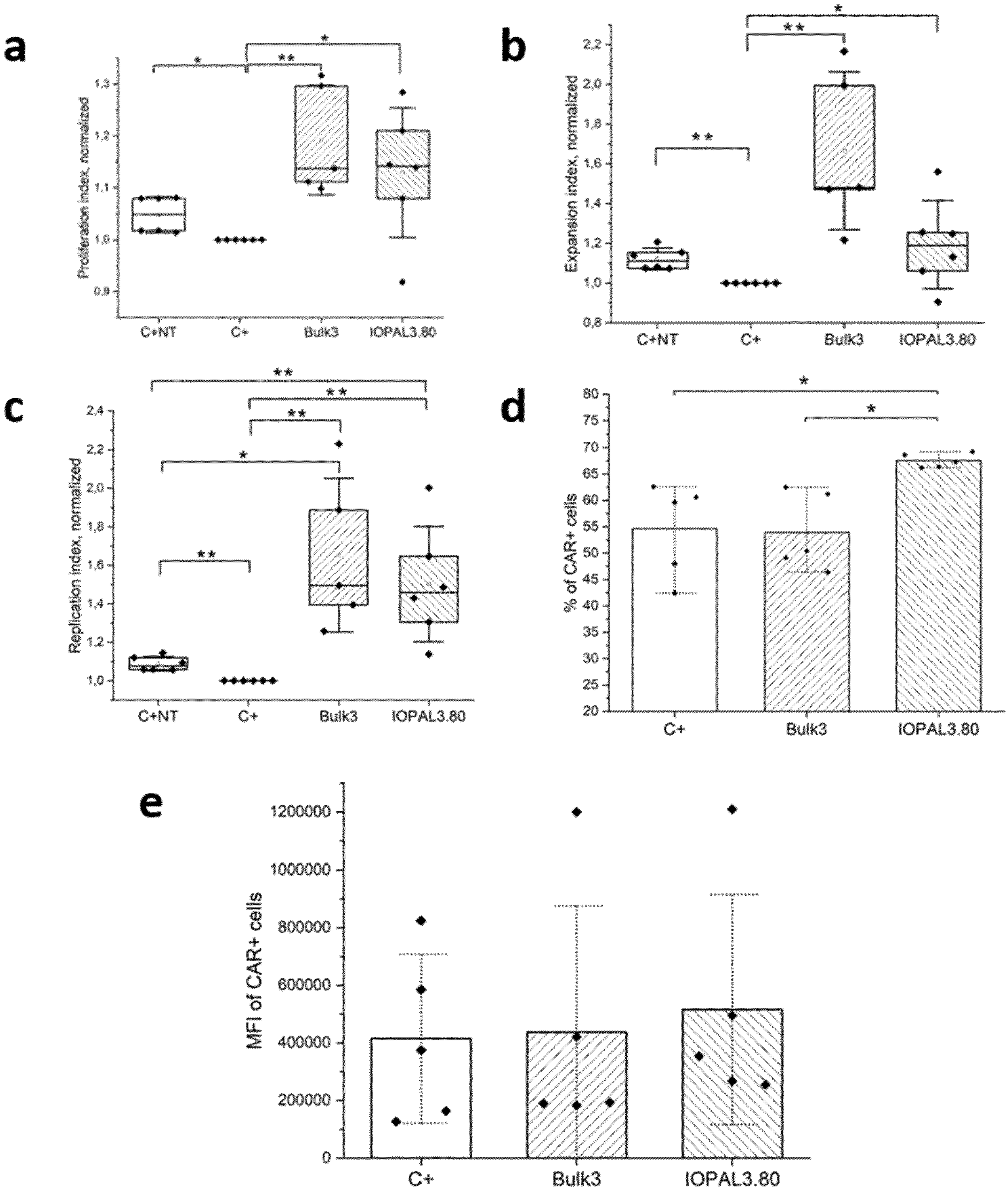
a
Proliferation index, normalized
C+NT
C+
Bulk3
IOPAL3.80
b
Expansion index, normalized
C+NT
C+
Bulk3
IOPAL3.80
c
Replication index, normalized
C+NT
C+
Bulk3
IOPAL3.80
d
% of CAR+ cells
C+
Bulk3
IOPAL3.80
e
MFI of CAR+ cells
C+
Bulk3
IOPAL3.80

FIG. 3

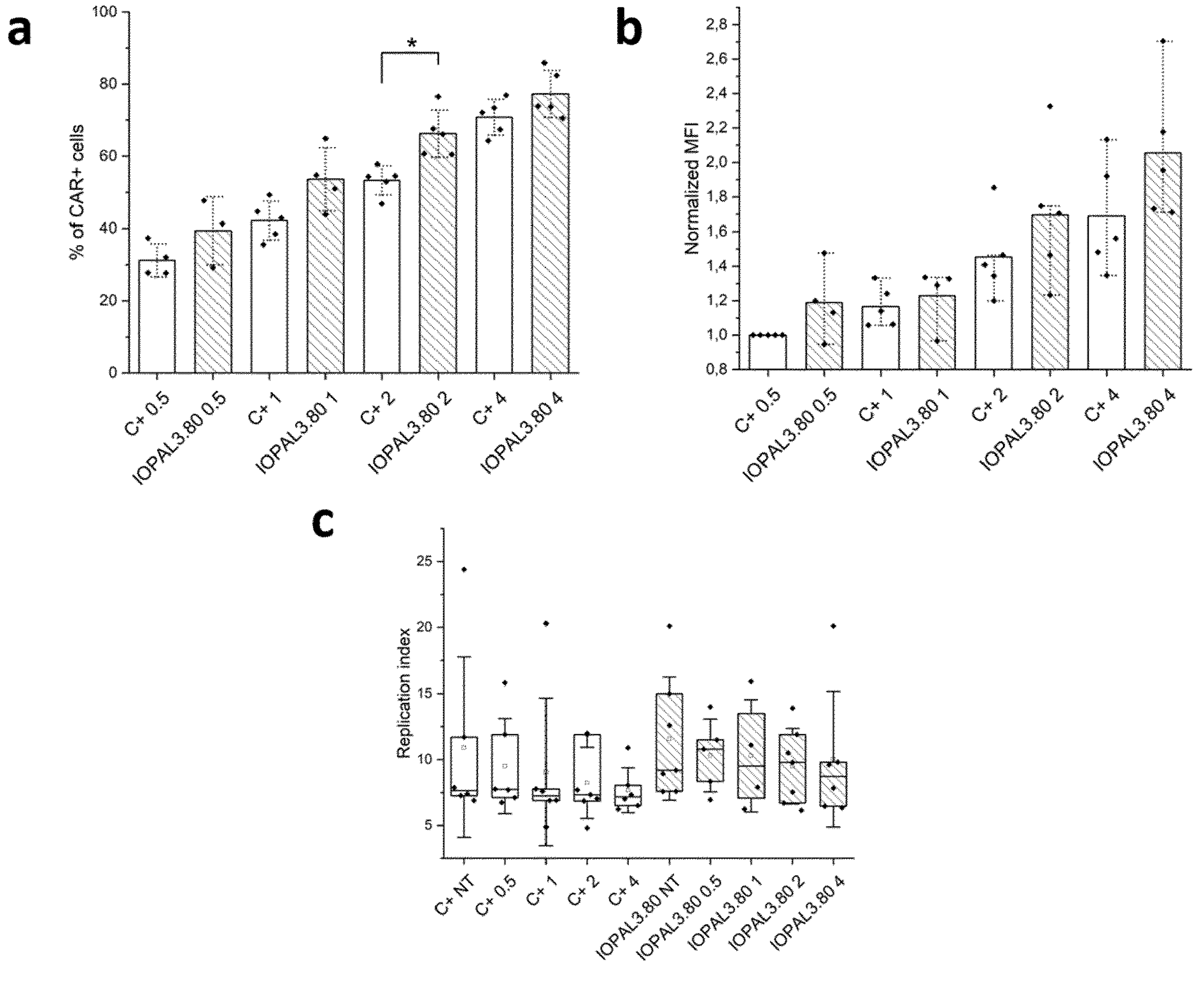
a
100
80
60
40
20
0
% of CAR+ cells
*
C+ 0.5
IOPAL3.80 0.5
C+ 1
IOPAL3.80 1
C+ 2
IOPAL3.80 2
C+ 4
IOPAL3.80 4
b
2,8
2,6
2,4
2,2
2,0
1,8
1,6
1,4
1,2
1,0
0,8
Normalized MFI
C+ 0.5
IOPAL3.80 0.5
C+ 1
IOPAL3.80 1
C+ 2
IOPAL3.80 2
C+ 4
IOPAL3.80 4
c
25
20
15
10
5
Replication index
C+ NT
C+ 0.5
C+ 1
C+ 2
C+ 4
IOPAL3.80 NT
IOPAL3.80 0.5
IOPAL3.80 1
IOPAL3.80 2
IOPAL3.80 4

FIG. 4

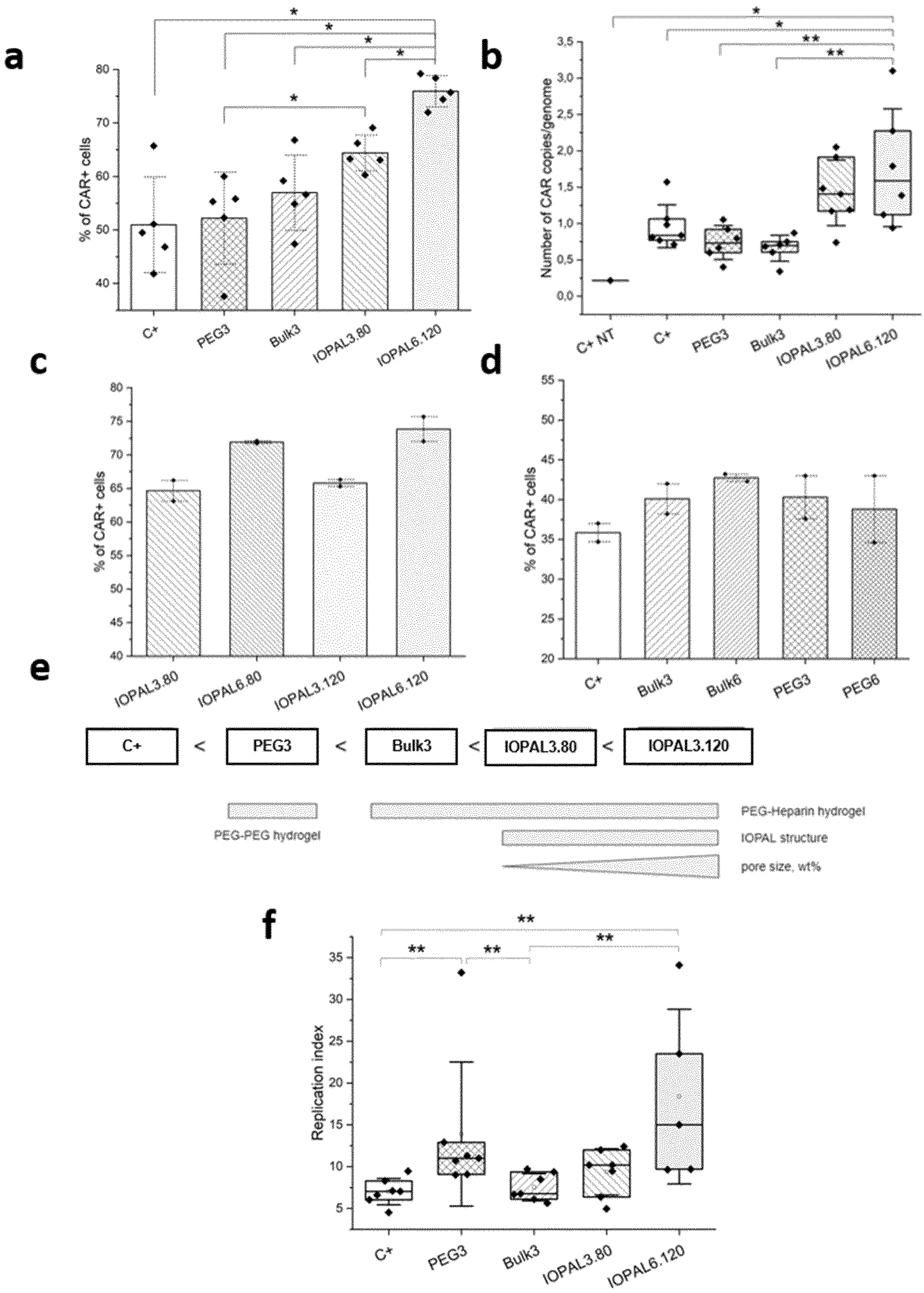
a
b
c
d
e
f
% of CAR+ cells
Number of CAR copies/genome
Replication index
C+ NT
C+
PEG3
PEG6
Bulk3
Bulk6
IOPAL3.80
IOPAL6.80
IOPAL3.120
IOPAL6.120
PEG-Heparin hydrogel
PEG-PEG hydrogel
IOPAL structure
pore size, wt%

FIG. 5

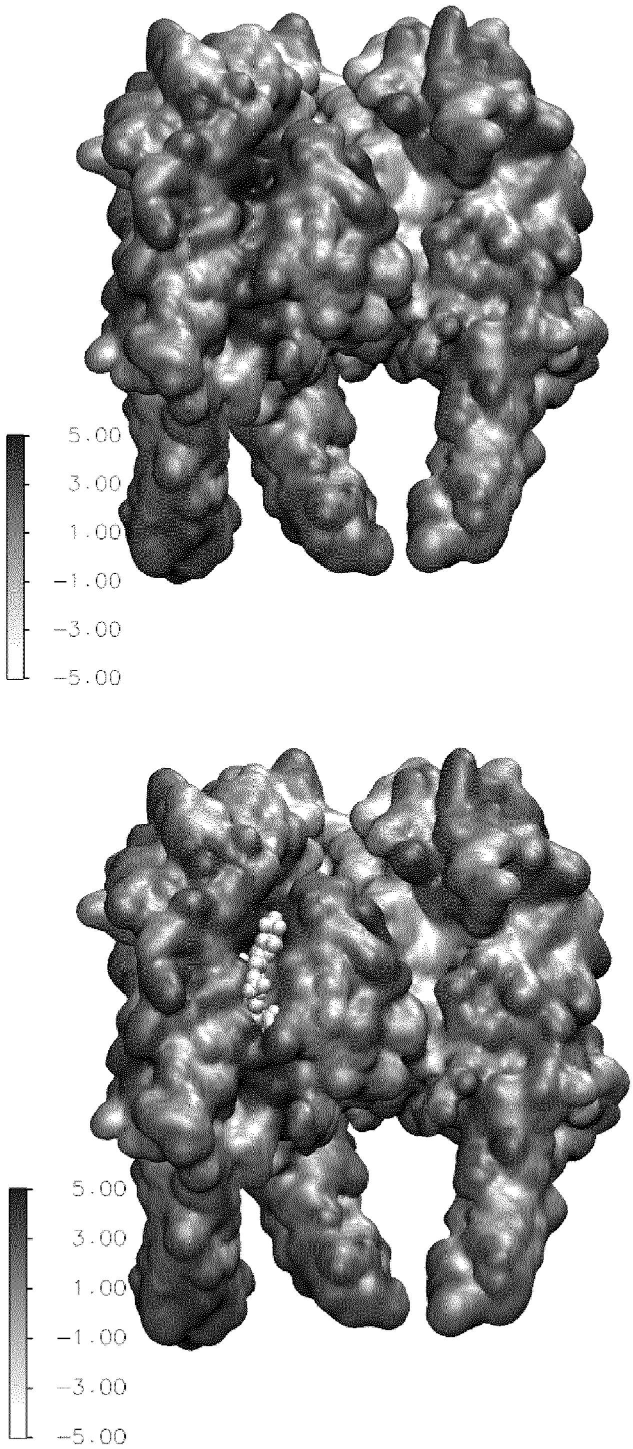


FIG. 6

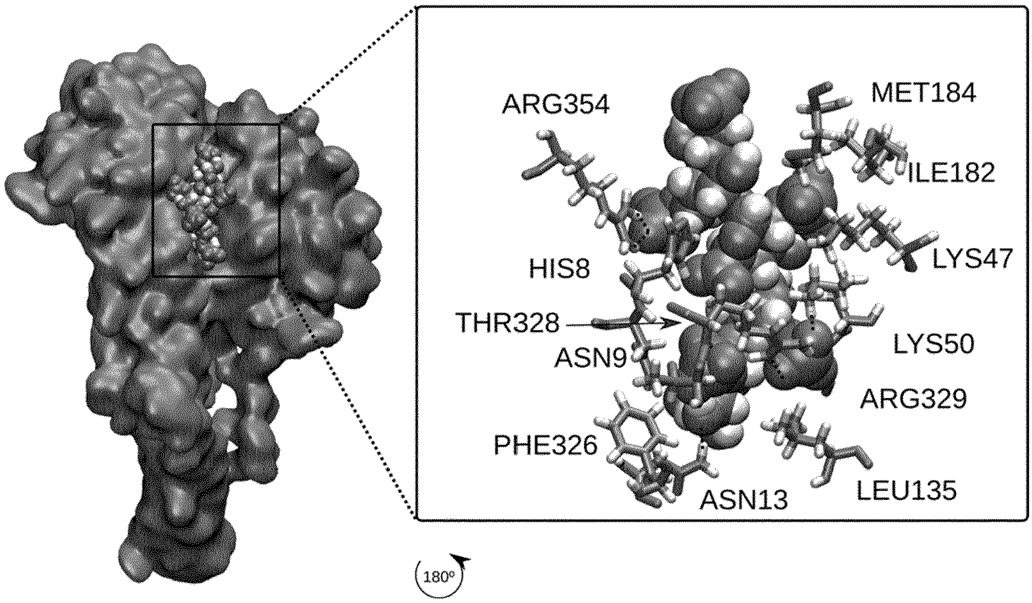
ARG354
MET184
ILE182
LYS47
HIS8
THR328
LYS50
ASN9
ARG329
PHE326
ASN13
LEU135
180º

FIG. 6 cont.

Europäisches Patentamt
European Patent Office
Office européen des brevets

# EUROPEAN SEARCH REPORT

Application Number
EP 24 38 2629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 252 789 A1 (CONSEJO SUPERIOR INVESTIGACION [ES]) 4 October 2023 (2023-10-04) * paragraphs [0021], [0051], [0055] - [0056], [0060] - [0061], [0100] * ----- | 1-15 | INV. A61L27/14 C12N5/00 C12N5/0783 |
| A | SANTOS FABIÃO ET AL: "Enhanced human T cell expansion with inverse opal hydrogels", BIOMATERIALS SCIENCE, vol. 10, no. 14, 1 January 2022 (2022-01-01), pages 3730-3738, XP093219886, GB ISSN: 2047-4830, DOI: 10.1039/D2BM00486K * page 3731, left-hand column, last paragraph * * page 3731, paragraphs 2.1, 2.2, 3.2, 3.3 - page 3735 * ----- | 1-15 | |
| A | WO 2021/234141 A1 (CONSEJO SUPERIOR INVESTIGACION [ES] ET AL.) 25 November 2021 (2021-11-25) * page 38, paragraph 1.2.1 * * page 37, paragraph 1.1 * * page 48, paragraph 1.6 * ----- | 1-15 | TECHNICAL FIELDS SEARCHED (IPC) A61L C12N |
| A | EDUARDO PÉREZ DEL RÍO ET AL: "CCL21-loaded 3D hydrogels for T cell expansion and differentiation", BIOMATERIALS, vol. 259, 13 August 2020 (2020-08-13), page 120313, XP055736611, AMSTERDAM, NL ISSN: 0142-9612, DOI: 10.1016/j.biomaterials.2020.120313 * paragraphs [2.1.2], [2.1.4]; figures 3, 4 * ----- -/-- | 1-15 | |

The present search report has been drawn up for all claims

2

| Place of search | Date of completion of the search | Examiner |
|---|---|---|
| Munich | 31 October 2024 | Petri, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 38 2629

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NIE T ET AL: "Production of heparin-containing hydrogels for modulating cell responses", ACTA BIOMATERIALIA, ELSEVIER, AMSTERDAM, NL, vol. 5, no. 3, 1 March 2009 (2009-03-01), pages 865-875, XP025990683, ISSN: 1742-7061, DOI: 10.1016/J.ACTBIO.2008.12.004 [retrieved on 2009-02-25] * abstract * ----- | 1-15 | |
| A | WO 2020/206128 A1 (UNIV JOHNS HOPKINS [US]) 8 October 2020 (2020-10-08) * paragraph [0117]; example 2 * ----- | 1-15 | |
| A | WO 2021/055658 A1 (UNIV CALIFORNIA [US]) 25 March 2021 (2021-03-25) * example 16 * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 31 October 2024 | Petri, Bernhard |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 24 38 2629

This annex lists the patent family members relating to the patent documents cited in the above-mentioned European search report. The members are as contained in the European Patent Office EDP file on
The European Patent Office is in no way liable for these particulars which are merely given for the purpose of information.

31-10-2024

| Patent document cited in search report | Publication date | Patent family member(s) | Publication date |
|---|---|---|---|
| EP 4252789 A1 | 04-10-2023 | EP 4252789 A1 | 04-10-2023 |
| | | WO 2023186794 A1 | 05-10-2023 |
| WO 2021234141 A1 | 25-11-2021 | EP 3913048 A1 | 24-11-2021 |
| | | EP 4153727 A1 | 29-03-2023 |
| | | US 2023201114 A1 | 29-06-2023 |
| | | WO 2021234141 A1 | 25-11-2021 |
| WO 2020206128 A1 | 08-10-2020 | US 2022168442 A1 | 02-06-2022 |
| | | WO 2020206128 A1 | 08-10-2020 |
| WO 2021055658 A1 | 25-03-2021 | CA 3152381 A1 | 25-03-2021 |
| | | EP 4031159 A1 | 27-07-2022 |
| | | US 2022403026 A1 | 22-12-2022 |
| | | US 2023250172 A1 | 10-08-2023 |
| | | WO 2021055658 A1 | 25-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3913048 A1
- EP 4252789 A1

### Non-patent literature cited in the description


- **GUEDAN, S. et al.** *Annu. Rev. Immunol.*, 2018, vol. 37, 145-171
- **WONG, W. K. et al.** *Eng. Regener.*, 2021, vol. 2, 70-81
- **ISSER, A. et al.** *Biomaterials*, 2021, vol. 268, 120584
- **TRICKETT, A.** ; **KWAN, Y. L. T**. *J. Immunol. Methods*, 2003, vol. 275, 251-255
- **PÉREZ DEL RIO, E. et al.** *Biomaterials*, 2020, vol. 259, 120313
- **SANTOS, F. et al.** *Biomater. Sci.*, 2022, vol. 10, 3730-3738
- **DULL, T. et al.** *J. Virol.*, 1998, vol. 72, 8463-8471
- **ROCHE, S.** *Cell. Mol. Life Sci.*, 2008, vol. 65, 1716-1728
- **FINCK, A. V.** ; **BLANCHARD, T.** ; **ROSELLE, C. P.** ; **GOLINELLI, G.** ; **JUNE, C. H.** Engineered cellular immunotherapies in cancer and beyond. *Nat. Med.*, 2022, vol. 28, 678-689
- **PÉREZ DEL RÍO, E. et al.** *Biomaterials*, 2020, vol. 259, 120313
- **BALDWIN, A. D.** ; **KIICK, K. L.** *Polym. Chem.*, 2013, vol. 4, 133-143
- **ZUIDEMA, J. M. et al.** *J. Biomed. Mater. Res. Part B*, 2014, vol. 102, 1063-1073, https://doi.org/-doi:10.1002/jbm.b.33088
- **CASTELLA, M. et al.** *Molecular Therapy Methods & Clinical Development*, 2019, vol. 12, 134-144
- **PÉREZ DEL RIO**. *Biomaterials*, 2020, vol. 259, 120313
- **PEINELT, A. et al.** *Front. Immunol.*, 2022, vol. 13, 830773
- **HUMPHREY, W.** *J. Mol. Graph.*, 1996, vol. 14, 33-38
- **PETTERSEN, E. F. et al.** *J. Comput. Chem.*, 2004, vol. 25, 1605-1612